(19) Europäisches Patentamt

European Patent Office

Office européen des brevets

(11) Publication number: **0 139 872**
**A1**

(12) **EUROPEAN PATENT APPLICATION**

(21) Application number: **84108469.2**

(22) Date of filing: **18.07.84**

(51) Int. Cl.⁴: **A 61 M 5/14**

(30) Priority: **18.08.83 US 524278**

(43) Date of publication of application:
**08.05.85 Bulletin 85/19**

(84) Designated Contracting States:
**BE DE FR GB IT**

(71) Applicant: **ABBOTT LABORATORIES**
**14th Street and Sheridan Road**
**North Chicago, Illinois 60064(US)**

(72) Inventor: **Gross, James Robert**
**1080 Independence Drive**
**Bartlett Illinois(US)**

(74) Representative: **Modiano, Guido et al,**
**MODIANO, JOSIF, PISANTY & STAUB Modiano &**
**Associati Via Meravigli, 16**
**I-20123 Milan(IT)**

(54) Flashback indicating system.

㊼ A magnifying element (13) is provided in conjunction
with a cannula hub (50) to afford an enlarged visual image of
the contents in the passageway (16) of the cannula hub (50).
More specifically, magnifying elements (13) are employed in
conjunction with a transparent cannula hub (50) to enlarge the
flashback of blood through a cannula (10) to indicate proper
placement of a cannula (10) into a blood vessel of a patient.

Fig. 1.

EP 0 139 872 A1

Croydon Printing Company Ltd.

## FLASHBACK INDICATING SYSTEM

### Background of the Invention

This invention relates generally to an improved flashback chamber. More specifically, this invention relates to a flashback chamber providing an enhanced visual indication of blood flow thereby allowing accurate placement of a catheter into a blood vessel.

The extraction of blood or the administration of medications directly into a blood vessel through a catheter requires exact placement of the end of a catheter into the vein or artery of a patient. Typically, a cannula is inserted into the bore of a catheter such that the pointed end of the cannula extends from the end of the catheter so it may be used to pierce the skin and the wall of the blood vessel. Once the pointed end of the cannula disappears beneath the surface of the skin there is only one sure method of telling when the pointed, open end of the cannula is within a vein or artery of a patient. This single method is to visualize the backflow or flashback of blood through the hollow portion of the cannula into the hub of the cannula. This indication of flashback will indicate to health care personnel that the end of the catheter is within a blood vessel of a patient. Once the near wall of a blood vessel has been punctured time becomes extremely important as continuous travel of the pointed end of the cannula over the very short distance between the walls of a blood vessel will cause puncture of the distant wall of the blood vessel and improper placement of the end of the catheter. Decreasing the time required for recognition of proper catheter placement will improve the quality and efficiency of treatment rendered by health care personnel by reducing the number of improper catheter placements.

Flashback indicators of the type concerned with in this invention are described in the Gewecke U.S.

0138972

Patent No. 2,868,200, in the Vcelka U.S. Patent No. 4,364,383 and in the McFarland U.S. Patent No. 4,365,630. Nowhere in the prior art is there provided a flashback or patency check device which includes a magnifying element to provide an enhanced visual indication that a cannula has properly been positioned in a vein or artery.

It is therefore an advantage of this invention to provide a flashback indicating system that will afford timely and positive recognition of proper placement of a cannula and catheter in a blood vessel.

Summary of the Invention

Described herein is a flashback indicating system comprising a transparent or translucent cannula hub body and flashback chamber onto which is placed a transparent magnifying element or lens-like surface such that the contents of the flashback chamber are magnified for health care personnel. This magnification allows for a quicker and larger visualization of the blood flashback into the flashback chamber. The lens itself may be any shape which provides an enlarged image to the observer. The magnifying element may extend the full length of the flashback chamber, in which case it also may provide an improved grip on the cannula hub.

Description of the Drawings

FIGURE 1 is a view in perspective of the flashback indicating system.

FIGURE 2 is a top plan view of the flashback indicating system of FIGURE 1.

FIGURE 3 is a side view in vertical section of the flashback indicating system of FIGURE 1.

FIGURE 4 is a sectional view of the flashback indicating system taken at line 4-4 in FIGURE 3 showing the effect of the magnification.

FIGURES 5 - 7 are views in perspective and side elevation depicting the operation of the flashback indicating system of FIGURE 1.

FIGURE 8 is a perspective view of an alternative embodiment of the flashback indicating system wherein a fin is mounted on the body portion of the flashback indicating system.

FIGURE 9 is a perspective view of an alternative embodiment of the flashback indicating system wherein a cylindrical magnifier is formed on the body portion.

## Description of the Embodiments

A better understanding of the present flashback indicating system will be accomplished by reference to the drawings wherein:

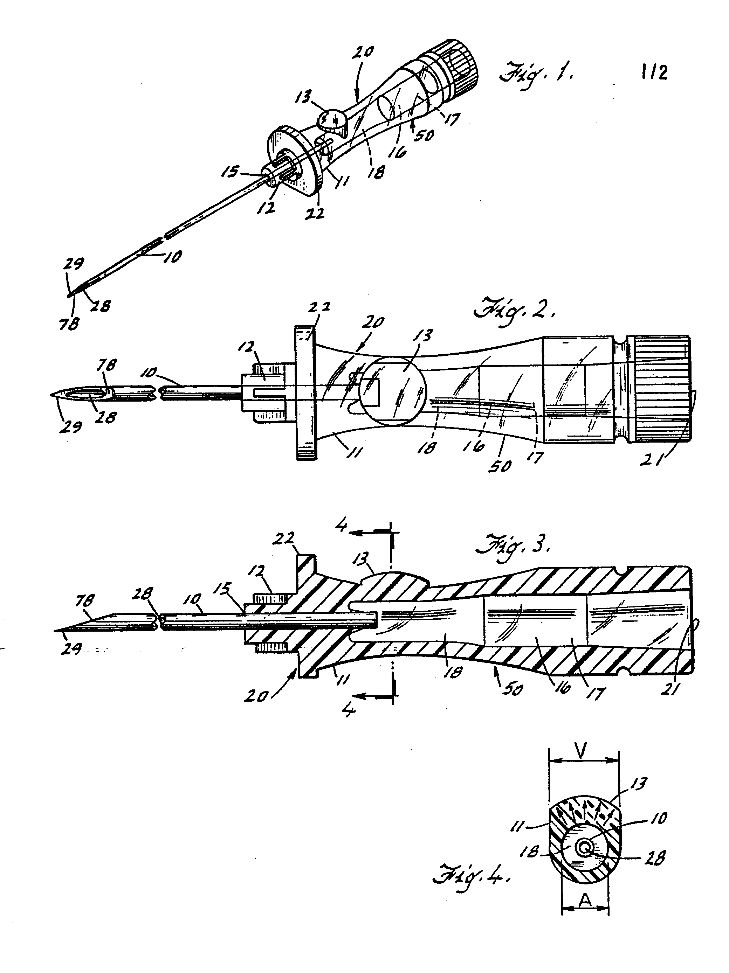

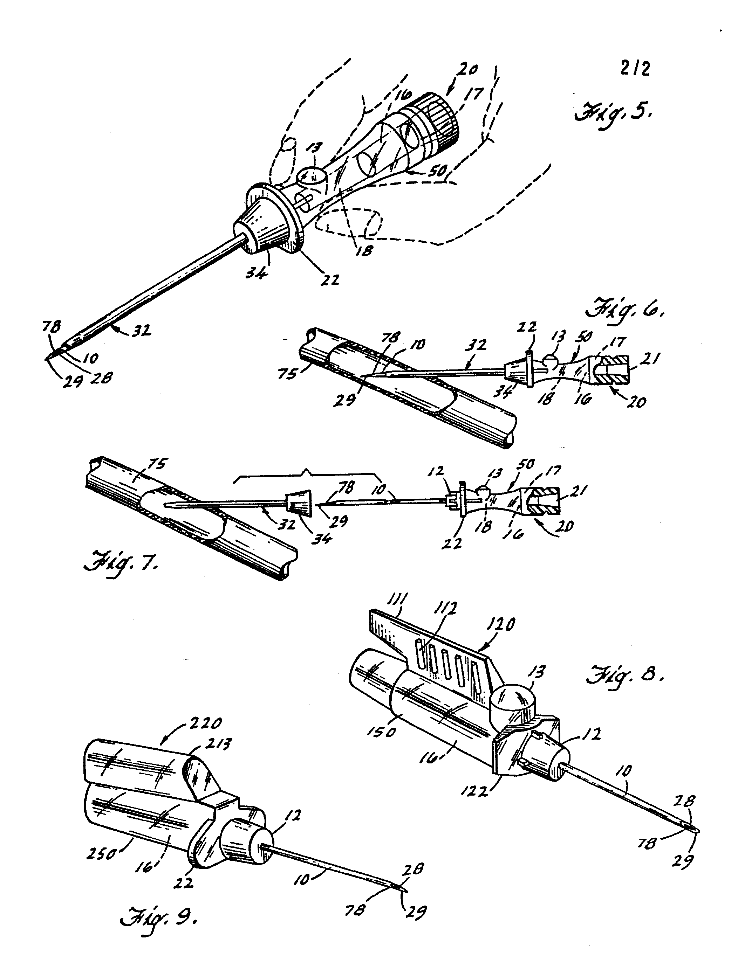

FIGURES 1 and 2 illustrate the flashback indicating system, generally 20 as it is received by health care personnel. The hollow cannula 10 is mounted through an end wall or small diameter portion 12 by being integrally molded such as at 15 through small diameter portion 12 of hub body generally 50. A chamber 16 within hub body 50 is in-fluid communication with the bore 28 of cannula 10. The large diameter body portion 11 of hub body 50 contains chamber 16. Chamber 16 has two portions: an indicating section 18 and a receiving section 17. A transparent lens-like magnifying element 13 is formed over transparent large diameter body portion 11 and indicating section 18. Finger stop 22 provides a surface for application of force and separation of the open end of the cannula 10 from contact with the fingers of the operator.

FIGURES 3 and 4 illustrate the two operative sections 17, 18 of chamber 16. The indicating section 18 is in fluid communication with the bore 28 of cannula 10. Magnifying element 13 is situated in order to give an enlarged visual indication of anything within indicating section 18. Receiving section 17 provides a receptacle for the blood once it has passed through indicating section 18. Hydrophobic filter 21 is mounted at the end of chamber 16 distal from cannula 10.

FIGURES 5-7 indicate the operation of the flashback indicating system 20 with catheter and hub assembly 32 and will be discussed in more detail later.

FIGURES 8-9 illustrate alternative embodiments wherein the same numerals have been employed to designate components having the same function, construction and relative location as in the embodiment of FIGURES 1-7.

In the embodiment of FIGURE 8 generally 120 a fin 111 has been added to hub body 150 over chamber 16. Finger stop 122 is shown as being squared rather than rounded as in the embodiment of FIGURES 1-7.

In the embodiment of FIGURE 9 generally 220 the magnifying element 213 is shown as a cylinder which covers the entire length of hub body 250.

### Operation

The flashback indicating system 20 of FIGURE 1 may be packaged separately or together with a catheter and hub assembly generally 32 (FIGURE 5). Finger stop 22 provides a surface upon which axial force may be exerted by thumb and forefinger (shown in phantom lines) to effect venipuncture. The use of a finger stop 22 allows the catheter and hub assembly 32 to remain sterile while being inserted into a blood vessel by preventing finger contact with the catheter hub 34. When the pointed end 29 of a hollow cannula 10 pierces the wall of a vein or artery 75 as shown in FIGURE 6, a portion of the blood contained within the vein or artery 75 flows through the hollow cannula 10 toward chamber 16. When this backflow of blood or 'flashback' first enters chamber 16 the magnifying element 13 provides an enhanced visual indication of the presence of blood in indicating section 18 by providing an image whose apparent width is the dimension V when the actual width is the dimension A (FIGURE 4). This larger image quickly and positively gives an indication that the tip 29 of the cannula 10 is within a blood vessel 75. The blood coming into chamber 16 passes through indicating section 18 into receiving section 17. The air contained with the chamber 16 which is displaced by the blood. This displaced air exits chamber 16 through hydrophobic membrane 21. Once this visual indication is received, health care personnel may then withdraw the flashback indicating system 20 from catheter and hub assembly 32 as shown in FIGURE 7. Fluid collection or insertion means (not shown) may now be attached to catheter and hub assembly 32. Once used, the low cost of the flashback indicating system 20 will allow for disposal.

In the FIGURE 8 embodiment the fin 111 may be used to assist in providing rotational orientation of the flashback indicating system 120. If desired ridges 112 may be added to the fin 111 to assist in providing a surface receptive of finger pressure.

In the FIGURE 9 embodiment a cylindrical magnifying element 213 is fabricated over hub body 250. This cylindrical magnifying element 213 enables health care personnel to observe an enhanced visual indication of the entire contents of chamber 16.

The preferred material for composing the magnifying element 13 and large diameter lens-like body portion 11 is a transparent material such as acrylonitrile. However, clear PVC or polypropylene, or butadiene-styrene could be used alternatively. It should be pointed out that if desired the large diameter body portion 11 which is not positioned beneath lens element 13 can be translucent. Standard injection molding techniques may be used to fabricate hub body 50, 150 or 250. The cannula 10 made of stainless steel may be molded into the transparent or translucent hub body or inserted after the hub body 50, 150 or 250 has been molded. The cannula is placed in hub body 50, 150 or 250 so that the bevel 78 is on the same side of cannula 10 as magnifying element 13. In this position magnifying element 13 not only provides an enhanced visual image of flashback but also provides an indication to health care personnel of the orientation of bevel 78 on cannula 10. Hydrophobic filter 21 may be made of Tyvek or eliminated entirely if leakage of blood through the flashback indicator system 20, 120 or 220 can be tolerated in a particular health care situation.

It will thus now be seen that through the present invention there is provided a flashback indicating system 20, 120 or 220 which can be packaged and assembled, then utilized with a minimum number of steps. The plastic portions of the flashback indicator system 20, 120 or 220 can be molded without the utilization of expensive or sophisticated molding techniques or materials. All of the components can be fabricated from relatively inexpensive materials, and once used, the flashback indicating system 20, 120 or 220 is disposable.

The foregoing invention can now be practiced by those skilled in the art. Such skilled persons will know that the invention is not necessarily restricted to the particular embodiments presented herein. The scope of the invention is to be defined by the terms of the following claims as given meaning by the preceding description.

CLAIMS:

1. A flashback indicating system comprising:

a cannula hub body defining a chamber;

a hollow pointed cannula secured to said hub body and in fluid communication with said chamber;

at least a section of said hub body being transparent; and

a magnifying element operatively associated with said translucent section of said hub body;

whereby said magnifying element enlarges the image of any fluid flowing into said chamber and provides an enlarged visual indication of fluid flow through said hub body.

2. A flashback indicating system comprising:

a cannula;

a cannula hub body defining a small diameter portion and a large diameter portion wherein said small diameter portion is constructed and arranged to receive a section of said cannula and said large diameter portion defines a chamber;

at least a portion of said large diameter portion being translucent; and

a magnifying element operatively associated with said translucent section of said large diameter portion of said cannula hub body whereby said magnifying element enlarges the image of any fluid flowing into said chamber and provides an enlarged visual indication of fluid flow through said translucent cannula hub body.

3. The flashback indicating system as defined in Claim 1 wherein the entire cannula hub body is translucent.

4. The flashback indicating system as defined in Claim 2 wherein said larger diameter portion of said translucent cannula hub body contains finger stops.

5. The flashback indicating system as defined in Claim 2 wherein finger stops are placed between said smaller diameter portion and said larger diameter portion of said translucent cannula hub body.

6. The flashback indicating system as defined in Claim 1 wherein said magnifying element is hemispherical in shape.

7. The flashback indicating system as defined in Claim 1 wherein said magnifying element is cylindrical in shape.

8. The flashback indicating system as defined in Claim 2 wherein said magnifying element is the same length as the translucent section of said cannula hub.

9. The flashback indicating system as defined in Claim 1 wherein said magnifying element is hemispherical in shape and further having a fin extending from said larger portion of said cannula hub.

10. A flashback system for indicating the presence of a catheter in a blood vessel system comprising:

a cannula hub body defining a small diameter portion and a large diameter portion wherein said small diameter portion is constructed and arranged to receive a section of said cannula and said large diameter portion defines a chamber;

finger stops places between said smaller diameter portion and said larger diameter portion of said cannula hub body;

a catheter constructed and arranged to surround said cannula leaving said sharpened tip exposed;

a catheter hub body connected to said catheter which surrounds said small diameter portion of said cannula hub body and further constructed and arranged to be in contact with said finger stops;

at least a portion of said larger diameter portion being transparent;

a magnifying element operatively associated with said translucent section of said large diameter portion of said cannula hub body; whereby when said cannula is inserted into a blood vessel said magnifying element enlarges the image of the flashback of blood into said chamber thus indicating the proper placement of the catheter into the blood vessel of a patient so that said cannula may be withdrawn from said catheter and catheter hub when said enlarged visual indication of the flashback of blood appears.

Fig. 1.                    112

Fig. 2.

Fig. 3.

Fig. 4.

212

Fig. 5.

Fig. 6.

Fig. 7.

Fig. 8.

Fig. 9.

# EUROPEAN SEARCH REPORT

| | DOCUMENTS CONSIDERED TO BE RELEVANT | | EP 84108469.2 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | US - A - 4 269 186 (J.C. LOVELESS et al.)<br>    * Totality *<br>    -- | 1-5 | A 61 M 5/14 |
| A | US - A - 3 859 998 (J.J. THOMAS et al.)<br>    * Totality *<br>    -- | 1-4 | |
| A | GB - A - 2 049 432 (B.B. ASBELL)<br>    * Totality *<br>    ---- | | |

**TECHNICAL FIELDS SEARCHED (Int. Cl.4)**

A 61 B 17/00

A 61 M 5/00

The present search report has been drawn up for all claims

| Place of search | Date of completion of the search | Examiner |
|---|---|---|
| VIENNA | 23-01-1985 | LUDWIG |